(19) **Europäisches Patentamt / European Patent Office / Office européen des brevets**

(11) **EP 2 760 414 B1**

(12) **EUROPEAN PATENT SPECIFICATION**

(45) Date of publication and mention of the grant of the patent:
**19.12.2018 Bulletin 2018/51**

(21) Application number: **12756509.1**

(22) Date of filing: **12.09.2012**

(51) Int Cl.:
*A61K 8/02* (2006.01)     *A61K 8/04* (2006.01)
*A61K 8/19* (2006.01)     *A61K 8/20* (2006.01)
*A61K 8/26* (2006.01)     *A61Q 15/00* (2006.01)

(86) International application number:
**PCT/EP2012/067787**

(87) International publication number:
**WO 2013/045270 (04.04.2013 Gazette 2013/14)**

(54) **ANTIPERSPIRANT COMPOSITIONS AND METHOD FOR REDUCING PERSPIRATION**

SCHWEISSHEMMENDE ZUSAMMENSETZUNGEN UND VERFAHREN ZUR SCHWEISSHEMMUNG

COMPOSITIONS ANTITRANSPIRANTES ET PROCÉDÉ POUR DIMINUER LA TRANSPIRATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.09.2011 EP 11183152**
**19.12.2011 EP 11194272**

(43) Date of publication of application:
**06.08.2014 Bulletin 2014/32**

(73) Proprietors:
• **Unilever PLC**
**London, Greater London EC4Y 0DY (GB)**
Designated Contracting States:
**CY GB IE MT**
• **Unilever N.V.**
**3013 AL Rotterdam (NL)**
Designated Contracting States:
**AL AT BE BG CH CZ DE DK EE ES FI FR GR HR HU IS IT LI LT LU LV MC MK NL NO PL PT RO RS SE SI SK SM TR**

(72) Inventors:
• **BAKER, Michael, Richard**
**Bebington**
**Wirral**
**Merseyside CH63 3JW (GB)**
• **FLETCHER, Neil, Robert**
**Bebington**
**Wirral**
**Merseyside CH63 3JW (GB)**
• **FRANKLIN, Kevin, Ronald**
**Bebington**
**Wirral**
**Merseyside CH63 3JW (GB)**
• **SHAFRAN, Kirill**
**Bebington**
**Wirral**
**Merseyside CH63 3JW (GB)**

(74) Representative: **Whaley, Christopher**
**Unilever PLC**
**Unilever Patent Group**
**Colworth House**
**Sharnbrook**
**Bedford, Bedfordshire MK44 1LQ (GB)**

(56) References cited:
**EP-A1- 1 974 716     WO-A2-2011/076569**
**US-A- 5 955 065**

• **DATABASE WPI Week 200903 Thomson Scientific, London, GB; AN 2009-A58216 XP002670238, -& JP 2008 303123 A (AKAHO KASEI KK) 18 December 2008 (2008-12-18)**
• **"Cosmetics and Drugs- Medicinal Mineral Salts ED - Bennett H", 1 January 2004 (2004-01-01), TWO THOUSAND FORMULAS, RECIPES & TRADE SECRETS: THE CLASSIC "DO-IT-YOURSELF" BOOK OF PRACTICAL EVERYDAY CHEMI, FERAL HOUSE, PAGE(S) 73, XP009171485, ISBN: 0-922915-95-4**

Remarks:
   The file contains technical information submitted after the application was filed and not included in this specification

Remarks:
   The file contains technical information submitted after the application was filed and not included in this specification

**Description**

The present invention is in the field of cosmetic compositions, in particular antiperspirant compositions and their use in reducing perspiration.

A variety of antiperspirant compositions have been marketed for many years. They serve to reduce perspiration, particularly following application to the surface of the body. Such compositions are typically considered cosmetic products, although certain countries do classify the active ingredients most commonly used in such compositions as pharmaceutical agents. The compositions are most commonly applied to the underarm regions of the human body.

The active ingredients conventionally used in antiperspirant compositions are astringent chlorohydroxide salts of aluminium and/or zirconium. These active ingredients are synthetic in origin, prepared in chemical plants and generally involving relatively advanced chemical processing steps. Such processing is not only expensive, but can also have significant environmental impact in terms of energy consumption.

Consumers are increasingly desirous of applying only "natural" ingredients and treatments to their body. Synthetic ingredients, in particular "active" ingredients, are often considered unsuitable for such application by consumers. There are a number of natural ingredients available that deliver some degree of deodorancy benefit when applied to the surface of the human body, but these ingredients are typically not capable of delivering a significant antiperspirancy benefit, i.e., they do not suppress perspiration to an extent that consumers would find acceptable. Hence, there is a problem in achieving good antiperspirancy using active ingredients that are natural ingredients.

Alum salts have been disclosed as suitable for use in a range of deodorant compositions and, indeed, such products have been marketed.

US 6,139,824 (L'Oreal, 2000) discloses the use of potassium alum in water-in-oil emulsions for deodorising the body. This patent also references several other publications in which potassium alum is used in aqueous and aqueous/ethanol solutions and in suspension sticks.

EP 1,974,716 A (Sara Lee, 2007) and WO 08/120976 (Sara Lee, 2008) disclose cosmetic compositions, for instance deodorant compositions, comprising at least partially dehydrated aluminium sulphate and a carrier liquid other than water.

Crystal Spring Ltd. offer or have offered a range of natural deodorants based upon the deodorising effect of potassium alum.

Green Bear UK Ltd. offer or have offered a crystal alum deodorant stick.

US 5,534,246 (Helen Curtis, 1996) discloses water-in-oil emulsion antiperspirant compositions in which alum salts are optional components; however no formulations containing alum salts are exemplified.

US 133,430 (John Gamgee, 1872) discloses the manufacture of a deodorising powder by mixing/grinding together aluminium sulphate (sulphate of alumina or "alum") and calcium chloride.

Other publications, such as US 5,955,065 (Gillette, 1999), have described the use of water soluble calcium salts to enhance the performance of conventional antiperspirant actives. The chemistry described in such publications involves the enhancement of peaks 3 and 4 on the HPLC trace of such antiperspirant actives. The species responsible for these peaks are not generated in the methods described herein and the chemistry behind the present invention is entirely different (*vide infra*).

WO 2011/076569 discloses antiperspirant compositions comprising an alum salt.

An objective of the present invention is to provide effective antiperspirant compositions the manufacture of which involves relatively low cost and relatively little environmental impact. In addition, the method and products of the invention may be seen as having good "natural" credentials, involving natural antiperspirant ingredients or at least naturally-derived antiperspirant ingredients.

A further objective of the present invention is to provide high efficacy antiperspirant compositions.

A further objective of the present invention is to provide a highly effective method of reducing perspiration and it is a particular objective that said method does not involve the use of synthetic aluminium and/or zirconium chlorohydroxide antiperspirant actives such as aluminium chlorohydrate.

In an aspect of the present description, there is provided a method for reducing perspiration comprising the application to the surface of the human body of an alum salt and calcium chloride.

In a first aspect of the present invention, there is provided an antiperspirant product comprising a cosmetically acceptable liquid carrier material, an alum salt and calcium chloride, characterised in that the alum salt and calcium chloride are formulated or contained in a manner that prevents or restricts chemical interaction between these two components prior to their application, according to claim 1.

In another aspect of the present description, there is provided a method of manufacture of an antiperspirant composition comprising the bringing together an alum salt and calcium chloride, characterised in that the alum salt is reduced in water content prior to combining with the calcium chloride.

The method for reducing perspiration described herein is for reducing perspiration from the surface of the human body, in particular from the underarm areas and the feet and especially from the underarm areas, otherwise known as the axillae.

The method may generally be considered a cosmetic method and products used in achieving the method, cosmetic products. That being said, the method can be extremely effective and may be also used to treat the medical condition of extreme sweating known as hyperhidrosis.

The method typically involves topical application of an alum salt and calcium chloride directly to the surface of the human body. In an alternative embodiment, an alum salt and calcium chloride may be applied indirectly to the surface of the human body, for example by application of said salts onto a wipe which is in turn applied to the surface of the human body.

The method may involve concurrent or sequential application of the salts to the surface of the body.

The method typically involves the application of one or both salts from a cosmetically acceptable liquid carrier material. Carrier materials suitable for this purpose are described in more detail below.

In certain preferred embodiments, the method involves application of both salts from a single composition. In this method the salts are prevented from premature interaction by formulating them in a manner that prevents or restricts chemical interaction between them prior to their application. One means of doing this is to minimise the water content of the composition (*vide infra*).

When the alum salt and calcium chloride are applied to the surface of the human body, whether from the same or different compositions, it is hypothesised that fluids derived from the sweat glands at least partially dissolve and hence mobilise the salts, allowing them to interact and thereby deliver a good antiperspirancy benefit.

The antiperspirant product typically used in delivery of the described method comprises an alum salt and calcium chloride, but it is crucial that these components are formulated or contained in a manner that reduces or prevents their physico-chemical interaction prior to their application.

In products according to the present invention, the alum salt and the calcium chloride may be prevented from physical interaction by any convenient means. In a simple form of product, the alum salt and the calcium chloride may be formulated in separate compositions and co-applied, either sequentially or at the same time. In such products, the product may comprise instruction to the consumer that both compositions must be applied to the same portion of the human skin, particularly when the product is not packaged to require simultaneous application of the compositions.

In a preferred embodiment, the alum salt and the calcium chloride are included in the same composition. In such compositions, the salts are prevented from premature interaction by formulating them in a manner that prevents or restricts chemical interaction between them prior to their application. A preferred method of doing this is to formulate them as dried powders into an anhydrous composition, that is to say into a composition having a very low level of free water (*vide infra*). The term "dried powder" should be understood to include both crystalline and amorphous states of matter. Such powders have a water content that is reduced from that of the most hydrated natural salt of the particular salt being used. More is said concerning preferred "dried powders" in the paragraphs described preferred alum salts and preferred calcium chloride salts.

The term "anhydrous" should be understood to mean having less than 2% by weight of free water; "free water" being water other than the water of hydration associated with any particular component. Preferably, anhydrous compositions have less than 1% by weight free water and more preferably less than 0.5%.

It is preferred that anhydrous compositions have a total water content (including water of hydration associated with components therein) of less than 10% by weight, and more preferably less than 5%.

The term alum salt as used in the present description means aluminium sulphate (sometimes called "alum") or any double sulphate of aluminium and a univalent metal ion selected from potassium, sodium, or ammonium. It does not include alum salts that are double sulphates of a univalent metal and a trivalent metal other than aluminium, such as chromium (III) or iron (III).

Alum salts for use in the present invention are potassium alum, ammonium alum, sodium alum and aluminium sulphate. That is to say:

$$M(i)Al(SO_4)_2 \text{ or } Al_2(SO_4)_3$$

wherein M(i) is $K^+$, $Na^+$, $NH_4^+$ or mixture thereof.

Preferred alum salts are ammonium and potassium alum, in particular potassium alum.

Preferred alum salts have a reduced content of water, that is to say, they are at least partially dehydrated. They may alternatively be described as dried powders (*vide supra*). It has been found that use of such salts in compositions also comprising calcium chloride improves ease of formulation and/or leads to improved storage stability for said compositions. Reducing the water content of the alum serves as a means for restricting chemical interaction between it and the calcium chloride prior to their application to the skin.

Potassium alum dodecahydrate has been found to be particularly difficult to formulate with calcium chloride; however, reducing its water content by 25% or greater can lead to acceptable compositions. In general, preferred alum salts for use in present invention have a water content of less than 35% by weight. Particularly preferred alum salts have a water content of less than 28% by weight and especially preferred alum salts have a water content of less than 20% by weight. When water is present, it is typically present as water of hydration.

The alum salt used in the present invention is typically milled to give it a reduced particle size. In preferred embodiments, the particle size distribution of the alum salt is such that its D50 is less than 75 microns and more preferably less than 50 microns. The particle size distribution of the alum salt is preferably such that less than 5% and more preferably less than 1% by weight of the particles have a particle size of greater than 120 microns.

The particle size distribution of the alum salt may advantageously be measured using a light scattering method on a Malvern Mastersizer 2000. The powder is dispersed in silicone fluid (DC245) and the results are analysed assuming a particle refractive index of 1.55 and imaginary refractive index of 0.001.

The calcium chloride used in the present invention may be anhydrous or hydrated, such as calcium chloride dihydrate, although anhydrous calcium chloride is preferred in many embodiments. Preferably, the calcium chloride is a dried powder (*vide supra*). The calcium chloride preferably has a water content of 25% or less, more preferably less than 15%, and most preferably less than 8% by weight. When water is present, it is typically present as water of hydration.

The water content of the calcium chloride is particularly important when it is formulated with alum salt. In compositions comprising both calcium chloride and alum salt, it is essential that the calcium chloride has a water content of 25% or less, unless there is some other means for restricting chemical interaction between it and the alum salt prior to their application to the skin. Suitable calcium chloride salts for such compositions include calcium chloride dihydrate and anhydrous calcium chloride, with anhydrous calcium chloride being preferred. It should be noted, however, than anhydrous calcium chloride as obtained from some suppliers can include up to about 14% by weight of water of hydration.

The calcium chloride used in the present invention is typically milled to give it a reduced particle size. In preferred embodiments, the particle size distribution of the calcium chloride is such that its D50 is less than 100 microns, more preferably less than 75 microns and most preferably less than 50 microns. The particle size distribution of the calcium chloride is preferably such that less than 5% and more preferably less than 1% by weight of the particles have a particle size of greater than 120 microns.

The particle size distribution of the alum salt may advantageously be measured using a light scattering method on a Malvern Mastersizer 2000. The powder is dispersed in silicone fluid (DC245) and the results are analysed assuming a particle refractive index of 1.55 and imaginary refractive index of 0.001.

Central to the present invention is the timely triggering of the following chemical reaction:

$$KAl(SO_4)_2 + 2CaCl_2 \rightarrow 2CaSO_4\downarrow + KCl + AlCl_3$$

Or

$$Al_2(SO_4)_3 + 3CaCl_2 \rightarrow 3CaSO_4\downarrow + 2AlCl_3$$

In the top equation, the potassium ion ($K^+$) may be substituted by sodium ($Na^+$) or ammonium ($NH_4^+$).

The stoichiometry of the above equations requires one mole of alum to two moles of calcium chloride in the first and one mole of alum to three moles of calcium chloride in the second. These equations set the basis for the preferred ratios of these components in compositions comprising both of these components. In such compositions, the molar quantity of calcium chloride exceeds the molar quantity of alum salt. It is also preferred that the quantity of calcium chloride at least matches that stoichiometrically required by the above equations, relative to the amount and type of alum present. This means that it is preferred that the molar ratio of calcium chloride to alum salt is at least 2:1.

In compositions comprising calcium chloride and sodium, potassium or ammonium alum as the major alum salt present, the molar ratio of calcium chloride to alum salt is preferably from 1:1 to 5:1, more preferably from 3:2 to 3:1, and most preferably about 2:1.

In compositions comprising calcium chloride and aluminium sulphate as the major alum salt present, the molar ratio of calcium chloride to alum salt is preferably from 2:1 to 6:1, more preferably from 5:2 to 4:1, and most preferably about 3:1.

It is important to the present invention that the reaction indicated above only occurs to a minimal extent before the components are delivered to the surface of the human skin. Premature reaction results in a physical state of matter which tends not to deliver the desired benefits; indeed, it is commonly extremely difficult to even apply said matter to the desired location.

The chemical reaction involved in the present invention may only occur when the ions making up the reactants have sufficient mobility. In certain preferred embodiments of the present invention, this mobility typically arises when the reacts dissolve in aqueous body fluids found on the surface of the human body. Magnesium chloride is ineffective when used instead of calcium chloride because of the much greater water solubility of magnesium sulphate compared with calcium sulphate.

Other components may also be included in compositions used in accordance with the invention.

A component included is a cosmetically acceptable liquid carrier material. Compositions preferably comprise the carrier material at a level of from 20% to 90%, or more preferably from 30% to 85% of the weight of the composition, excluding any volatile propellant present.

Such carrier materials are typically liquid, by which is meant liquid at ambient temperature and pressure (20°C and 1 atmosphere, for the purposes of this specification). Preferably, such carrier substances are anhydrous, as described hereinabove, especially when co-formulated with the alum salt and calcium chloride. Preferably, carrier materials contain less than 2%, more preferably less than 1% and most preferably less than 0.5% by weight free water.

Preferred liquid carrier materials also perform an addition function; particularly preferred liquid carrier materials are emollients and/or masking oils.

Preferred carrier materials are hydrophobic. Hydrophobic liquid carrier materials particularly suitable for use are liquid silicones, that is to say, liquid polyorganosiloxanes. Such materials may be cyclic or linear, examples include Dow Corning silicone fluids 344, 345, 244, 245, 246, 556, and the 200 series; Union Carbide Corporation Silicones 7207 and 7158; and General Electric silicone SF1202. Alternatively, non-silicone hydrophobic liquids may be used. Such materials include mineral oils, hydrogenated polyisobutene, polydecene, paraffins, isoparaffins of at least 10 carbon atoms, ether oils such as PPG-14 butyl ether, and aliphatic or aromatic ester oils (e.g. triethyl hexanoin, isopropyl myristate, lauryl myristate, isopropyl palmitate, diisopropyl sebecate, diisopropyl adipate, or $C_8$ to $C_{18}$ alkyl benzoates). Particularly preferred carrier materials are ester oils, in particular C12-15 alkyl benzoate, available as Finsolv TN from Finetex.

In embodiments in which the alum salt is not co-formulated with the calcium chloride, hydrophilic liquid carrier materials may advantageously be employed. Such materials include water and polar organic solvents. When water is used as a carrier material for the alum salt and/or the calcium chloride, it is strongly preferred that the alum salt and the calcium chloride are applied from independent compositions. This ensures that premature interaction does not occur between the components (*vide supra*). Polar organic solvents that may be employed include $C_1$-$C_4$ monohydric alcohols, for example ethanol and isopropanol, and polyols, for example propylene glycol, dipropylene glycol, glycerol, polyethylene glycol, and $C_2$-$C_8$ 1,2-alkanediols like 1,2-hexanediol.

Additional antiperspirant actives may also be included.

The total amount of antiperspirant actives, including alum salt and calcium chloride, incorporated in a composition is preferably from 0.5-50%, particularly from 1 to 30% and especially from 2% to 26% of the weight of the composition.

Antiperspirant actives used in addition to the alum salt and calcium chloride combination are often selected from astringent active salts, including in particular aluminium, zirconium and mixed aluminium/zirconium salts. Preferred additional antiperspirant actives are aluminium, zirconium and aluminium/zirconium halides and halohydrate salts, such as chlorohydrates.

Suitable aluminium halohydrates are defined by the general formula $Al_2(OH)_xQ_y.wH_2O$ in which Q represents chlorine, bromine or iodine, x is variable from 2 to 5 and x + y = 6 while $wH_2O$ represents a variable amount of hydration. Especially effective aluminium halohydrate salts are known as activated aluminium chlorohydrates and are made by methods known in the art.

Suitable zirconium actives are represented by the empirical general formula: $ZrO(OH)_{2n-nz}B_z.wH_2O$ in which z is a variable in the range of from 0.9 to 2.0 so that the value 2n-nz is zero or positive, n is the valency of B, and B is selected from the group consisting of chloride, other halide, sulphamate, sulphate and mixtures thereof.

Antiperspirant complexes based on the above-mentioned astringent aluminium and/or zirconium salts can be employed. The complex often employs a compound with an amino acid, such as glycine.

The proportion of solid antiperspirant salt in a suspension composition normally includes the weight of any water of hydration and any complexing agent that may also be present in the solid active.

Additional deodorant actives may also be included. When employed, the level of incorporation is preferably from 0.01% to 3% and more preferably from 0.03% to 0.5% by weight. Preferred deodorant actives are those that are more efficacious than simple alcohols such as ethanol. Examples include quaternary ammonium compounds, like cetyltrimethylammonium salts; chlorhexidine and salts thereof; and diglycerol monocaprate, diglycerol monolaurate, glycerol monolaurate, and similar materials, as described in "Deodorant Ingredients", S.A.Makin and M.R.Lowry, in "Antiperspirants and Deodorants", Ed. K. Laden (1999, Marcel Dekker, New York). More preferred are polyhexamethylene biguanide salts (also known as polyaminopropyl biguanide salts), an example being Cosmocil CQ available from Arch Chemicals; 2',4,4'-trichloro,2-hydroxy-diphenyl ether (triclosan); and 3,7,11-trimethyldodeca-2,6,10-trienol (farnesol).

Other components particular to the type of composition in which the invention is used may also be included. Types of composition in which the invention may be used include, non-exclusively, sticks, soft solids, aerosols, and roll-ons.

Stick or soft solid compositions typically comprise one or more structurants or gellants, which serves to thicken the composition. Such thickeners, referred to as structurant systems, may be selected from those known in the art for such purpose. The present inventors have found the choice of structurants to be of particular importance when the alum salt and calcium chloride are included in the same composition. In such compositions, it has been found that particularly suitable structurant systems comprise:

1. stearyl alcohol as the major component, preferably in the presence of lesser amounts of polyethylene wax and hydrogenated castor oil; or

2. polyethylene wax as the major component, preferably in the presence of lesser amount of hydrogenated castor oil.

In general, structurant and gellants suitable for use in compositions according to the present invention may be classed as waxes or non-polymeric fibre-forming gellants.

"Waxes" may be defined as water-insoluble materials that are solid at 30°C and preferably also at 40°C. They may be selected from hydrocarbons, linear fatty alcohols, silicone polymers, esters waxes or mixtures thereof.

Examples of hydrocarbon waxes include paraffin wax, ozakerite, microcrystalline wax and polyethylene wax, the last named desirably having an average molecular weight of from 300 to 600 and advantageously from 350 to 525.

Linear fatty alcohols commonly contain from 14 to 40 carbon atoms and often from 16 to 24. In practice, most contain an even number of carbon atoms and many comprise a mixture of compounds, even those that are nominally a single one such as stearyl alcohol.

Silicone polymer waxes typically satisfy the empirical formula:-

$$1. \quad R\text{-}(SiMe_2\text{-}O\text{-})_x\text{-}SiMe_2R$$

in which x is at least 10, preferably 10 to 50 and R represents an alkyl group containing at least 20 carbons, preferably 25 to 40 carbons, and particularly having an average linear chain length of at least 30 carbons; or

$$2. \quad Y\text{-}(SiMe_2\text{-}O\text{-})_y(Si[OR']Me\text{-}O\text{-})_z\text{-}Y'$$

in which Y represents $SiMe_2$-O, Y' $SiMe_2$, R' an alkyl of at least 15 carbons preferably 18 to 22 such as stearyl, y and z are both integers, totalling preferably from 10 to 50.

Examples of ester waxes include esters of $C_{16}$-$C_{22}$ fatty acids with glycerol or ethylene glycol, which can be isolated from natural products or more conveniently synthesised from the respective aliphatic alcohol and carboxylic acid.

"Non-polymeric fibre-forming gellants" are capable of being dissolved in a water-immiscible blend of oils at elevated temperature and on cooling precipitating out to form a network of very thin strands that are typically no more than a few molecules wide. One particularly effective category of such thickeners comprises N-acyl aminoacid amides and in particular linear and branched N-acyl glutamic acid dialkylamides, such as in particular N-lauroyl glutamic acid di n-butylamide and N-ethylhexanoyl glutamic acid di n-butylamide and especially mixtures thereof. Such amido gellants can be employed in anhydrous compositions according to the present invention, if desired, with 12-hydroxystearic acid.

Other such non-polymeric fibre-forming gellants include 12-hydroxystearic acid amides, and amide derivatives of di- and tri-basic carboxylic acids as set forth in WO 98/27954, including notably alkyl N,N'dialkyl succinamides.

Further suitable structuring systems comprising non-polymeric fibre-forming gellants of this type are described in US 6,410,003, US 7,332,153, US 6,410,001, US 6,321,841, and US 6,248,312.

The structurant or gellant is often employed in the stick or soft solid composition at a concentration of from 1.5 to 30%. When a non-polymeric fibre-forming gellants is employed as the major component of the structuring system, its concentration is typically in the range of from 1.5 to 7.5% by weight for amido gellants or mixtures of them and for 5 to 15% for ester or sterol gellants. When a wax is employed as the major component of the structuring system, its concentration is usually selected in the range of from 10 to 30% by weight, and particularly from 12 to 24% by weight.

Other types of structurant or gellant disclosed in the prior art may alternatively be employed.

Aerosol compositions suitable for use in accordance with the invention are characterised by comprising a propellant, typically a liquefied hydrocarbon or halogenated hydrocarbon gases (particularly fluorinated hydrocarbons such as 1,1-difluoroethane and/or 1-trifluoro-2-fluoroethane) that have a boiling point of below 10°C and especially those with a boiling point below 0°C. It is especially preferred to employ liquified hydrocarbon gases, and especially $C_3$ to $C_6$ hydrocarbons, including propane, butane, isobutane, pentane and isopentane and mixtures of two or more thereof. Preferred propellants are isobutane, isobutane/propane, butane/propane and mixtures of propane, isobutane and butane.

Other propellants that can be contemplated include alkyl ethers, such as dimethyl ether or compressed non-reactive gasses such air, nitrogen or carbon dioxide.

The propellant is typically the major component of aerosol compositions, often comprising from 30 to 99% weight and preferably comprising from 50 to 95% by weight.

In certain preferred embodiments, aerosol compositions may also comprise a liquid carrier material other than the propellant. These may be selected as appropriate from those previously mentioned, hydrophobic liquid carrier materials being especially preferred.

In certain preferred embodiments, aerosol compositions may also comprise a suspending agent, for example, a hydrophobically modified clay, such as disteardimonium hectorite (Bentone 38V), ex Elementis, typically at from 0.1 to 1.5% by weight.

Propylene carbonate may also be advantageously employed in aerosol compositions used in accordance with the present invention, typically at from 0.001 to 0.1% by weight.

Roll-on compositions suitable for use in accordance with the invention are typically suspension products, in particular suspensions of alum salt and calcium chloride in an anhydrous liquid carrier material (*vide supra*), hydrophobic liquid carrier materials being preferred.

Roll-on compositions preferably comprise a suspending agent, for example, a hydrophobically modified clay, such as disteardimonium hectorite (Bentone 38V), ex Elementis, typically at from 0.5 to 3% by weight.

Roll-on compositions preferably comprise a particulate sensory modifier, for example finely divided clay such as Aerosil 200, ex Evonik Degussa, typically at from 0.01 to 0.5% by weight.

Certain sensory modifiers are further desirable components in the compositions of the invention. Such materials are preferably used at a level of up to 20% by weight of the composition. Emollients, humectants, volatile oils, non-volatile oils, and particulate solids that impart lubrication are all suitable classes of sensory modifiers. Examples of such materials include cyclomethicone, dimethicone, dimethiconol, isopropyl myristate, isopropyl palmitate, talc, finely-divided silica (e.g. Aerosil 200), particulate polyethylene (e.g. Acumist B18), polysaccharides, corn starch, C12-C15 alcohol benzoate, PPG-3 myristyl ether, octyl dodecanol, C7-C14 isoparaffins, di-isopropyl adipate, isosorbide laurate, PPG-14 butyl ether, glycerol, hydrogenated polyisobutene, polydecene, titanium dioxide, phenyl trimethicone, dioctyl adipate, and hexamethyl disiloxane.

In certain compositions, emulsifiers that are perfume solubilisers and/or wash-off agents are preferred additional components. Examples of the former include PEG-hydrogenated castor oil, available from BASF in the Cremaphor RH and CO ranges, preferably present at up to 1.5% by weight, more preferably 0.3 to 0.7% by weight. Examples of the latter include poly(oxyethylene) ethers.

In many embodiments of the invention, fragrance is a desirable additional component. Suitable materials include conventional perfumes, such as perfume oils and also include so-called deo-perfumes, as described in EP 545,556, for example. Levels of incorporation are preferably up to 5% by weight, particularly from 0.1% to 3.5% by weight, and especially from 0.5% to 2.5% by weight. The fragrance may also be added in an encapsulated form, release being triggered post-application by hydrolysis or shear on the surface of the human body.

Further additional components that may also be included are colourants and preservatives at a conventional level, for example $C_1$-$C_3$ alkyl parabens.

The method of manufacture of products of the invention may involve independent manufacture of a first composition comprising alum salt, a second composition comprising calcium chloride, and packaging of the compositions in such a manner as to enable both compositions to be applied to the same portion of the human skin, whether sequentially or (preferably) simultaneously.

When the invention involves use of a composition comprising both alum salt and calcium chloride, the method of manufacture of said composition typically comprises the alum salt being reduced in water content prior to mixing with the calcium chloride in a carrier material. In such methods, the alum salt is preferably reduced in water content to less than 35%, more preferably less than 28% and most preferably less than 20% by weight.

## Examples

The following examples illustrate certain specific embodiments of the invention and do not limit the scope of the invention. Examples according to the invention are indicated by numbers and comparative examples are indicated by letter. Examples indicated by both number and letter require co-application with another example in order to meet the requirements of the invention. All amounts indicated are percentages by weight, unless otherwise indicated.

The compositions indicated in Table 1 were prepared as follows. The oils [components (1) to (3)] were blended together at 90°C and the waxes [components (4) to (6)] were melted in with stirring. When the waxes were fully melted, the mixtures were cooled to 75-80°C and salt or salts selected from components (7) to (9) as indicated were added (calcium chloride first, when used) and well dispersed into the mixture. The mixtures were cooled to about 62°C and poured into stick barrels.

**Table 1 - Stick Compositions**

| Example: | 1 | 2 | 3 | 4 | A |
|---|---|---|---|---|---|
| **Component:** | | | | | |
| Silicone oil (1) | 39.5 | 41.1 | 50.5 | 39.5 | 46.15 |
| Ester oil (2) | 11.0 | 11.0 | 11.0 | 22.0 | 11.0 |
| Ether oil (3) | 11.0 | 11.0 | -- | -- | 11.0 |
| Stearyl alcohol (4) | 18.0 | 18.0 | 18.0 | 18.0 | 18.0 |
| Polyethylene wax (5) | 1.0 | 1.0 | 1.0 | 1.0 | 1.0 |
| Hydrogenated castor oil (6) | 3.5 | 3.5 | 3.5 | 3.5 | 3.5 |
| Dried potassium alum [14-18% $H_2O$] (7) | 9.35 | -- | 9.35 | 9.35 | 9.35 |
| Dried potassium alum [3% $H_2O$] (8) | -- | 7.75 | -- | -- | -- |
| Anh. calcium chloride (9) | 6.65 | 6.65 | 6.65 | 6.65 | -- |

(1) Cyclopentasiloxane, DC245, ex Dow Corning.
(2) C12-15 alkyl benzoate, Finsolv TN, ex Finetex.
(3) PPG-14 butyl ether, Fluid AP, exAmerchol.
(4) Lanette C18 Deo, ex Cognis.
(5) Performalene 400, molecular weight ca. 400, ex Alfa Chemicals.
(6) Castor wax MP80, ex Caschem.
(7) Raw material ex Sigma-Aldrich dried at 65°C and jet milled to give a particle size (D50) of 38-45 micron.
(8) Raw material ex Sigma-Aldrich dried at 200°C and hammer milled to give a particle size (D50) of less than 20 micron.
(9) Less than 4% water (by weight), ex Sigma-Aldrich, jet milled to give a particle size (D50) of 24 micron.

The antiperspirancy performance of Example 1 was compared with that of Comparative Example A in a direct head-to-head panel test as described below.

Test operators applied Example 1 (0.30g) to one axilla and Comparative Example A (0.30g) to the other axilla of each panellist. This was done once each day for three days. After the third application, panellists were requested not to wash under their arms for the following 24 hours.

24 hours after the third and final product application, the panellists were induced to sweat in a hot-room at 40°C (±2°C) and 40% (±5%) relative humidity, for 40 minutes. After this period, the panellists left the hot-room and their axillae were carefully wiped dry. Pre-weighed cotton pads were then applied to each axilla of each panellist and the panellists re-entered the hot-room for a further 20 minutes. Following this period, the pads were removed and re-weighed, enabling the weight of sweat generated to be calculated.

The sweat weight reduction (SWR) for each panellist was calculated as a percentage (% SWR) and the mean % SWR was calculated according to the method described by Murphy and Levine in "Analysis of Antiperspirant Efficacy Results", J. Soc. Cosmetic Chemists, 1991(May), 42, 167-197.

Example 1 was found to give a 19% greater mean SWR than Comparative Example A.

When a stick composition analogous to Examples 1 and 2 was attempted using potassium alum dodecahydrate (ex Sigma-Aldrich) instead of dried potassium alum, the stick did not form due to an unfavourable interaction between the alum salt and the calcium chloride.

When stick compositions analogous to Examples 1 and 2 were prepared using calcium chloride dihydrate [raw material described under Table 6 as component (14)] instead of anhydrous calcium chloride, the sticks produced were soft and crumbly, leading to a visually undesirable appearance. This problem did not manifest itself with the alternative stick compositions indicated in Table 6 as Examples 17 and 18 (*vide infra*).

Scale-up studies revealed that Examples 3 and 4 were superior to Examples 1 and 2, being more robust to processing conditions. Examples 1 and 2 became unacceptably soft when made at large (3kg) scale.

Compositions 5 and 6 indicated in Table 2 were prepared as follows. The oils [components (1) and (2)] were blended together at 95°C and the waxes [components (5) and (6)] were melted in with stirring at 90-95°C. When the waxes were fully melted, the mixtures were cooled to 85°C and salts selected from components (7) to (9) (calcium chloride first, when used) as indicated were added and well dispersed into the mixture. The mixtures were cooled to about 75°C and poured into stick barrels.

Compositions 7a and 7b indicated in Table 2 may be prepared by means analogous to those used to prepare Compositions 5 and 6. Application of both Composition 7a and 7b to the same axilla is required in order to obtain a significant underarm antiperspirancy benefit.

**Table 2 - Further Stick Compositions**

| Example: | 5 | 6 | 7a | 7b |
|---|---|---|---|---|
| **Component:** | | | | |
| Silicone oil (1) | To 100 | To 100 | To 100 | To 100 |
| Ester oil (2) | 30.0 | 30.0 | 30.0 | 30.0 |
| Polyethylene wax (5) | 15.0 | 15.0 | 15.0 | 15.0 |
| Hydrogenated castor oil (6) | 2.0 | 2.0 | 2.0 | 2.0 |
| Dried potassium alum [15.5% $H_2O$] (7) | 9.4 | -- | 9.4 | -- |
| Dried potassium alum [3% $H_2O$] (8) | -- | 7.8 | -- | -- |
| Anhydrous calcium chloride (9) | 6.7 | 6.7 | -- | 6.7 |
| (1), (2), and (5) to (9) as indicated under Table 1. | | | | |

The antiperspirancy efficacy of Example 5 and an analogous composition having the calcium chloride replaced by silicone oil (1) were compared, in separate tests, with that of a non-antiperspirant body spray using a method analogous to those used to compare Example 1 with Comparative Example A. Example 5 was found to give a 28% greater SWR than the analogous composition without the calcium chloride.

The antiperspirancy efficacy of Example 6 and an analogous composition having the calcium chloride replaced by silicone oil (1) were compared, in separate tests, with that of a non-antiperspirant body spray using a method analogous to those used to compare Example 1 with Comparative Example A. Example 6 was found to give a 27% greater SWR than the analogous composition without the calcium chloride.

Antiperspirant aerosol Examples 8 to 12 indicated in Table 3 were prepared as follows. The oil or oils [components (1) and (2)] were blended at ambient temperature with the suspending agent [component (10)], followed by the propylene carbonate and fragrance, each being added with shear. Salts selected from components (7) to (9) as indicated were then added (calcium chloride first) and well dispersed into the mixture. The resulting base compositions were placed in aerosol cans which were closed with a standard valve and valve cup and the liquefied propellant [component (11)] then added.

The antiperspirancy efficacy of Example 10 was compared with that of a non-antiperspirant body spray control in a test analogous to that used to compare Example 1 with Comparative Example A, except that the aerosol was dosed at approximately 2g per application (equivalent to a 2 second spray). In this test Example 5 was found to give a 23% greater SWR than the control. An analogous antiperspirancy test was performed to assess Example 12 and this product was found to give a 42% greater SWR than the control. When a further test was performed with a product analogous to Example 12, but with the calcium chloride replaced by silicone oil (1), a SWR only 11% greater than the control was observed, a non-significant difference at the 95% level.

Aerosol compositions analogous to Examples 10 and 11 were unsuccessfully attempted using ether oil (3) instead of ester oil (2). The resulting base compositions became warm and set solid within one hour of preparation.

**Table 3 - Aerosol Compositions**

| Example: | 8 | 9 | 10 | 11 | 12 |
|---|---|---|---|---|---|
| **Component:** | | | | | |
| Silicone oil (1) | 7.89 | 8.29 | 5.89 | 6.29 | 2.89 |
| Ester oil (2) | -- | -- | 2.0 | 2.0 | 2.0 |
| Propylene carbonate | 0.01 | 0.01 | 0.01 | 0.01 | 0.01 |
| Fragrance | 0.6 | 0.6 | 0.6 | 0.6 | 0.6 |
| Dried potassium alum [14-18% $H_2O$] (7) | 2.34 | -- | 2.34 | -- | 3.51 |
| Dried potassium alum [3% $H_2O$] (8) | -- | 1.94 | -- | 1.94 | -- |
| Anhydrous calcium chloride (9) | 1.66 | 1.66 | 1.66 | 1.66 | 2.49 |

(continued)

| Example: | 8 | 9 | 10 | 11 | 12 |
|---|---|---|---|---|---|
| Component: | | | | | |
| Suspending agent (10) | 0.5 | 0.5 | 0.5 | 0.5 | 0.5 |
| Propellant (11) | To 100 | To 100 | To 100 | To 100 | To 100 |
| (1), (2), and (7) to (9) as indicated under Table 1.<br>(10) Disteardimonium hectorite, Bentone 38V, ex Elementis.<br>(11) AP40, ex HARP. | | | | | |

The antiperspirant compositions indicated in Table 4 may be prepared by means analogous to those used to prepare Examples 8 to 12. Spray application of both Example 13a and 13b to the same axilla is required in order to obtain a significant underarm antiperspirancy benefit.

In an antiperspirancy test analogous to that used to test Examples 10 and 12, Example 14 gave a 48% greater SWR than the control.

### Table 4 - Further Aerosol Compositions

| Example: | 13a | 13b | 14 |
|---|---|---|---|
| Component: | | | |
| Silicone oil (1) | 5.89 | 5.89 | 10.6 |
| Ester oil (2) | 2.0 | 2.0 | 4.0 |
| Propylene carbonate | 0.01 | 0.01 | 0.10 |
| Fragrance | 0.6 | 0.6 | 1.0 |
| Dried potassium alum [14-18% $H_2O$] (7) | 2.34 | -- | 4.7 |
| Anh. calcium chloride (9)<br>Anh. calcium chloride (12) | --<br>-- | 1.66<br>-- | --<br>3.6 |
| Suspending agent (10) | 0.5 | 0.5 | 1.0 |
| Propellant (11) | To 100 | To 100 | |
| (1), (2), (7), and (9) to (11) as indicated under Table 3.<br>(12) Less than 7% water (by weight), *ex* Sigma-Aldrich, jet milled to give a particle size (D50) of 20-35 micron. | | | |

Roll-on composition 15 indicated in Table 5 may be prepared as follows. The oils [components (1), (2), and (13)], fragrance and the suspending agent are sheared for 10 minutes at 8000 rpm with a Silverson mixer and the propylene carbonate then slowly added, followed by the fumed silica, and the mixture sheared for a further 5 minutes. The salts [components (8) and (9)] are then added (calcium chloride first) and the mixture again sheared for 5 minutes. The homogeneous mixture is then transferred into a roll-on pack.

### Table 5 - Roll-on Compositions

| Example: | 15 | 16a | 16b |
|---|---|---|---|
| Component: | | | |
| Silicone oil (1) | To 100 | To 100 | To 100 |
| Ester oil (2) | 28.5 | 28.5 | 28.5 |
| Octyl dodecanol (12) | 0.2 | 0.2 | 0.2 |
| Suspending agent (10) | 1.5 | 1.5 | 1.5 |
| Propylene carbonate | 1.0 | 1.0 | 1.0 |
| Fragrance | 1.0 | 1.0 | 1.0 |

(continued)

| Example: | 15 | 16a | 16b |
|---|---|---|---|
| Component: | | | |
| Fumed silica (13) | 0.05 | 0.05 | 0.05 |
| Dried potassium alum [3% $H_2O$] (8) | 11.63 | -- | 11.63 |
| Anhydrous calcium chloride (9) | 9.98 | 9.98 | -- |
| (1), (2) and (7) to (10) as indicated under Table 1. (13) Eutanol G, *ex* Cognis. (14) Aerosil 200, *ex* Evonik Degussa. | | | |

Roll-on compositions 16a and 16b indicated in Table 5 may be prepared by means analogous to those described for prepare Example 15. Application of both Example 16a and 16b to the same axilla is required in order to obtain a significant underarm antiperspirancy benefit.

The stick compositions indicated in Table 6 were prepared by methods analogous to those used to prepare the examples illustrated in Table 2.

### Table 6 - Further Stick Compositions

| Example: | 17 | 18 | 19 | 20 |
|---|---|---|---|---|
| Component: | | | | |
| Silicone oil (1) | To 100 | To 100 | To 100 | To 100 |
| Ester oil (2) | 30.0 | 30.0 | 30.0 | 30.0 |
| Polyethylene wax (5) | 15.0 | 15.0 | 15.0 | 15.0 |
| Hydrogenated castor oil (6) | 2.0 | 2.0 | 2.0 | 2.0 |
| Dried potassium alum [15.5% $H_2O$] (7) | -- | -- | 9.4 | 14.1 |
| Dried potassium alum [3% $H_2O$] (8) | -- | 7.8 | -- | -- |
| Aluminium sulphate (15) | 5.1 | - | - | - |
| Anhydrous calcium chloride (9) | 5.0 | - | - | 10.0 |
| Calcium chloride dihydrate (16) | - | 8.8 | 8.8 | - |
| (1), (2), and (5) to (9) as indicated under Table 1. (15) Tai-ace S100, ex Taimei Chemicals Co Ltd., average particle size 8-10 microns, water content less than 1.5% by weight. (16) ex Sigma-Aldrich, found to have particles of predominately less than 100 microns by optical microscopy following processing. | | | | |

The Examples in Table 7 were also prepared by methods analogous to those used to prepare the Examples illustrated in Table 2. The indicated SWR values were obtained in tests analogous to those used for the assessment of Examples 5 and 6.

### Table 7 - Further Stick Compositions

| Example: | 21 | 22 | 23 | 24 |
|---|---|---|---|---|
| Component: | | | | |
| Silicone oil (1) | To 100% | To 100% | To 100% | To 100% |
| Ester oil (2) | 30.0 | 30.0 | 30.0 | 30.0 |
| Polyethylene wax (5) | 15.0 | 15.0 | 15.0 | 15.0 |
| Hydrogenated castor oil (6) | 2.0 | 2.0 | 2.0 | 2.0 |

(continued)

| Example: | 21 | 22 | 23 | 24 |
|---|---|---|---|---|
| Component: | | | | |
| Dried potassium alum [14-18% $H_2O$] (7) | 2.3 | 4.7 | 9.4 | 9.4 |
| Anhydrous calcium chloride [93%] (12) | 1.8 | 3.6 | 3.6 | 8.6 |
| Molar ratio calcium chloride : alum | 2:1 | 2:1 | 1:1 | 3:1 |
| **SWR (%)** | **25** | **44** | **36** | **55** |

All components as previously defined.

The SWR result for Examples 21 and 22 illustrate that significant antiperspirancy benefits can be achieved at relative low levels of alum salt and calcium chloride.

A comparison of the SWR result of Examples 23 and 24 reveals that a reasonable SWR can be achieved at a molar ratio of calcium chloride to alum salt of 1:1, but that this is much better when, at the same level of alum salt, the molar ratio of calcium chloride to alum salt is increased to 3:1.

## Claims

1.   An anhydrous antiperspirant composition comprising less than 2% by weight free water, a cosmetically acceptable liquid carrier material, a first salt that is an alum salt of formula $M(i)Al(SO_4)_2$ or $Al_2(SO_4)_3$ wherein M(i) is $K^+$, $Na^+$, $NH_4^+$ or mixture thereof and a second salt that is calcium chloride, **characterised in that** the first salt and the second salt are formulated or contained in a manner that prevents physical interaction between these two components prior to their application and wherein the composition has a molar ratio of calcium chloride to alum salt that is at least 1:1.

2.   An antiperspirant composition according to claim 1, wherein the alum salt is potassium aluminium sulphate.

3.   An antiperspirant composition according to claim 1 or claim 2, wherein the alum salt has a water content of less than 35% by weight, preferably less than 28% by weight and more preferably less than 20% by weight.

4.   An antiperspirant composition according to any of the preceding claims, wherein the calcium chloride has a water content of less than 15% and preferably less than 8% by weight.

## Patentansprüche

1.   Wasserfreie schweißhemmende Zusammensetzung, umfassend weniger als 2 Gewichts-% freies Wasser, ein kosmetisch verträgliches flüssiges Trägermaterial, ein erstes Salz, das ein Alaun-Salz der Formel $M(i)Al(SO_4)_2$ oder $Al_2(SO_4)_3$ ist, worin M(i) $K^+$, $Na^+$, $NH_4^+$ oder eine Mischung davon ist, und ein zweites Salz, das Calciumchlorid ist, **dadurch gekennzeichnet, dass** das erste Salz und das zweite Salz formuliert werden oder in einer Weise enthalten sind, dass eine physikalische Wechselwirkung zwischen diesen zwei Bestandteilen vor deren Auftragen verhindert wird, und wobei die Zusammensetzung ein Molverhältnis von Calciumchlorid zu Alaun-Salz aufweist, das mindestens 1:1 ist.

2.   Schweißhemmende Zusammensetzung nach Anspruch 1, wobei das Alaun-Salz Kaliumaluminiumsulfat ist.

3.   Schweißhemmende Zusammensetzung nach Anspruch 1 oder Anspruch 2, wobei das Alaun-Salz einen Wassergehalt von weniger als 35 Gewichts-%, vorzugsweise weniger als 28 Gewichts-% und bevorzugter weniger als 20 Gewichts-% aufweist.

4.   Schweißhemmende Zusammensetzung nach irgendeinem der vorhergehenden Ansprüche, wobei das Calciumchlorid einen Wassergehalt von weniger als 15 Gewichts-% und vorzugsweise weniger als 8 Gewichts-% aufweist.

**Revendications**

1. Composition d'antiperspirant anhydre comprenant moins de 2 % en masse d'eau libre, un matériau de support liquide cosmétiquement acceptable, un premier sel qui est un sel d'alun de formule $M(i)Al(SO_4)_2$ ou $Al_2(SO_4)_3$ où $M(i)$ est $K^+$, $Na^+$, $NH_4^+$ ou un mélange de ceux-ci et un second sel qui est le chlorure de calcium, **caractérisée en ce que** le premier sel et le second sel sont formulés ou contenus dans une manière qui évite une interaction physique entre ces deux constituants avant leur application et dans laquelle la composition présente un rapport molaire de chlorure de calcium à sel d'alun qui est d'au moins 1:1.

2. Composition d'antiperspirant selon la revendication 1, dans laquelle le sel d'alun est le sulfate de potassium aluminium.

3. Composition d'antiperspirant selon la revendication 1 ou la revendication 2, dans laquelle le sel d'alun présente une teneur en eau inférieure à 35 % en masse, de préférence inférieure à 28 % en masse et encore mieux inférieure à 20 % en masse.

4. Composition d'antiperspirant selon l'une quelconque des revendications précédentes, dans laquelle le chlorure de calcium présente une teneur en eau inférieure à 15 % et de préférence inférieure à 8 % en masse.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6139824 A
- EP 1974716 A
- WO 08120976 A
- US 5534246 A
- US 133430 A
- US 5955065 A
- WO 2011076569 A

- WO 9827954 A
- US 6410003 B
- US 7332153 B
- US 6410001 B
- US 6321841 B
- US 6248312 B
- EP 545556 A

**Non-patent literature cited in the description**

- Deodorant Ingredients. **S.A.MAKIN ; M.R.LOWRY.** Antiperspirants and Deodorants. Marcel Dekker, 1999

- **MURPHY ; LEVINE.** Analysis of Antiperspirant Efficacy Results. *J. Soc. Cosmetic Chemists,* May 1991, vol. 42, 167-197